(19) Europäisches Patentamt / European Patent Office / Office européen des brevets

(11) **EP 1 765 160 B1**

(12) **EUROPEAN PATENT SPECIFICATION**

(45) Date of publication and mention
of the grant of the patent:
**10.06.2015 Bulletin 2015/24**

(51) Int Cl.:
***A61B 5/053*** (2006.01)

(21) Application number: **05739888.5**

(22) Date of filing: **27.04.2005**

(86) International application number:
**PCT/KR2005/001216**

(87) International publication number:
**WO 2005/110218 (24.11.2005 Gazette 2005/47)**

(54) **APPARATUS FOR DETECTING ANOMALIES WITHIN BODY**

GERÄT ZUM NACHWEIS VON ANOMALIEN IM KÖRPER

APPAREIL DE DETECTION D'ANOMALIES CORPORELLES

(84) Designated Contracting States:
**DE FR GB**

(30) Priority: **13.05.2004 KR 2004033928**

(43) Date of publication of application:
**28.03.2007 Bulletin 2007/13**

(73) Proprietor: **University-Industry Cooperation
Group of KyungHee
University
Yongin,
Kyungki-do 449-701 (KR)**

(72) Inventors:
• **WOO, Eung Je
  Seongnam-si,
  Gyeonggi-do 463-743 (KR)**
• **SEO, Jin Keun
  Seocho-gu,
  Seoul 137-040 (KR)**
• **KWON, Ohin
  Yangcheon-gu,
  Seoul 158-751 (KR)**

(74) Representative: **Samson & Partner Patentanwälte
mbB
Widenmayerstraße 6
80538 München (DE)**

(56) References cited:
**US-A- 5 810 742          US-A1- 2002 183 645
US-A1- 2002 193 685      US-A1- 2003 004 432**

• **BERNHARD SCHOLZ: "Towards Virtual Electrical
Breast Biopsy: Space-Frequency MUSIC for
Trans-Admittance Data", IEEE TRANSACTIONS
ON MEDICAL IMAGING, IEEE SERVICE CENTER,
PISCATAWAY, NJ, US, vol. 21, no. 6, 1 June 2002
(2002-06-01), XP011076313, ISSN: 0278-0062**

## Description

Technical Field

The present invention relates to apparatuses for detecting a lesion, such as cancer or tumor, by using electric properties of a body.

Background Art

According to the annual report of the central cancer registry, it can be noted that there is ceaseless increase of breast cancers and colorectum cancers in the case of women. Particularly, the incidence rate of breast cancers has increased continuously to become a first place (16.1%) of women cancer incidence rates in 2001, and it is foreseen that it will increase continuously. In a case of Japan, the breast cancer incidence rate was 19.6% of all cancer incidence rates in 2001, and in a case of the USA, the breast cancer incidence rate was 37.3% of all cancer incidence rates in 2001.

The most preferable treatment is early examination, and treatment of the tumor before the tumor transfers to other part of the body. However, such an early diagnosis requires a positive and effective diagnosis method. Presently, efforts for the early diagnosis of the breast cancer are under progress actively by using various methods. For the early diagnosis, a variety of medical apparatus is used, such as apparatus of X-ray mammography, ultrasonography, thermography, MRI, CT, and so on. However, in the case of the X-ray mammography which is used the most widely, due to characteristics of the X-ray, the X-ray mammography has a disadvantage in that the diagnosis of young women who has a high density of a lobule-alveolar-system is difficult, and, furthermore, there is a hazard of the breast cancer caused by the X-ray used for the diagnosis. In the case of the ultrasonography, the ultrasonography has a disadvantage in that resolution is low, such that there are ceaseless discussion on the effectiveness of the ultrasonography, and application to the early diagnosis is difficult. In the case of the thermography which utilizes a point that cancerous tissue or inflammation has a temperature higher than a normal cell, it is difficult to diagnosis the breast cancer by using the thermography, because the thermography shows an image of temperatures of skin, mainly. MRI or CT requires high diagnosis cost and is difficult to take an image of the breast only.

Recently, there have been researches for detecting the breast cancer by using a phenomenon in which there is a three to ten times difference of conductivities between a normal tissue and a tumor tissue. Of the researches, T-Scan of the TransScan Medical Ltd. takes a method of application of a voltage and measurement of current, to present a transfer admittance map of the organic tissue. The measuring method of the T-Scan is identical to the frontal plane impedance camera which is the first electrical impedance tomography (EIT) of which research is started from early 1980s. That is, an electrostatic voltage is applied to a part of human body by using a surface electrode, currents from array electrodes attached to the other part of the human body are measured while the voltage of the array electrodes are maintained at the ground potential, and the measured currents are displayed as an image. The image is called as a transfer admittance image. The T-Scan presents the transfer admittance image, and the user determines a patient having a breast cancer by making subjective interpretation of the image. In other words, the diagnosis apparatus like the T-Scan fails to be provided with a function for analyzing data measured on a surface of the breast. Therefore, it is difficult for the T-Scan to make an accurate detection of a lesion because a diagnosis result is dependent on a visual determination of the user.

US 2003/004432 A1 discloses that an excitation may be applied as voltage and/or current signals in one or more frequencies, that different frequencies are used for different procedures, and how an indication of the type of anomaly is provided by the electrifying signals of different phases applied to the electrodes.

US 2002/193685 A1 discloses a system and method for accurately locating and tracking the position of a target, such as a tumor or the like, within a body.

US 2002/183645 A1 discloses an apparatus for breast examination comprising a sensing unit including a probe capable of sensing electrical signals from a surface of a breast. The apparatus does not attempt to determine the location of an anomaly but only to provide a general indication as to whether such an anomaly exists. For this, it applies electrical signals at a plurality of frequencies, measures response signals for each of the frequencies, and determines an indicator frequency at which the imaginary admittance reaches a local maximum. The indicator frequency is optionally used in generating a malignancy score.

US 5 810 742 A discloses an apparatus for aiding in the identification of tissue type for an anomalous tissue in an impedance image.

Disclosure of Invention

An object of the present invention is" to provide apparatus for detecting a lesion, which can make accurate detection of, not only existence of lesion like a breast cancer, but also a size of the lesion with reference to a transfer admittance data measured at a surface of a human body.

The object of the present invention can be achieved by providing an apparatus according to claim 1, for detecting a lesion including an electrode for applying a voltage to a part of a body, a probe having a plurality of sensing electrodes for sensing signals from an examining part of the body, and a control unit for determining a position and a size of a lesion in the body with reference to the signals sensed by the sensing electrodes.

The probe includes a plate for arranging the sensing electrodes at regular intervals thereon, and a metal frame around the plate for maintaining the sensing electrodes at a reference voltage.

The control unit determines a pinpoint of the lesion in a probe plane opposite to a center of the lesion with reference to the sensed signals, and calculates a distance from the center of the lesion to the pinpoint of the lesion in the probe plane for determining a position of the lesion. The control unit calculates a volume of the lesion with reference to an average of the sensed signals, a distance between the center of the lesion and the pinpoint of the lesion in the probe plane, an intensity of the signal sensed at the pinpoint of the lesion in the probe plane, for determining a size of the lesion.

The methods disclosed herein do not form part of the invention and are merely provided to illustrate the functions of the apparatus according to the invention.

Brief Description of Drawings

FIG. 1 illustrates a block diagram of an apparatus for detecting a lesion in accordance with a preferred embodiment of the present invention;
FIG. 2 illustrates a diagram showing a method for detecting a lesion by using an apparatus for detecting a lesion in accordance with a preferred embodiment of the present invention;
FIG. 3 illustrates a block diagram of the electrostatic voltage generator in FIG. 1;
FIG. 4 illustrates a diagram of a scan probe of the present invention;
FIGS. 5A, 5B, and 5C illustrate diagrams each showing a variation of a scan probe of the present invention;
FIG. 6 illustrates a block diagram of the current measuring unit in FIG. 1;
FIG. 7 illustrate diagrams showing images of measured parts by using the device for detecting lesion of the present invention.

Best Mode for Carrying Out the Invention

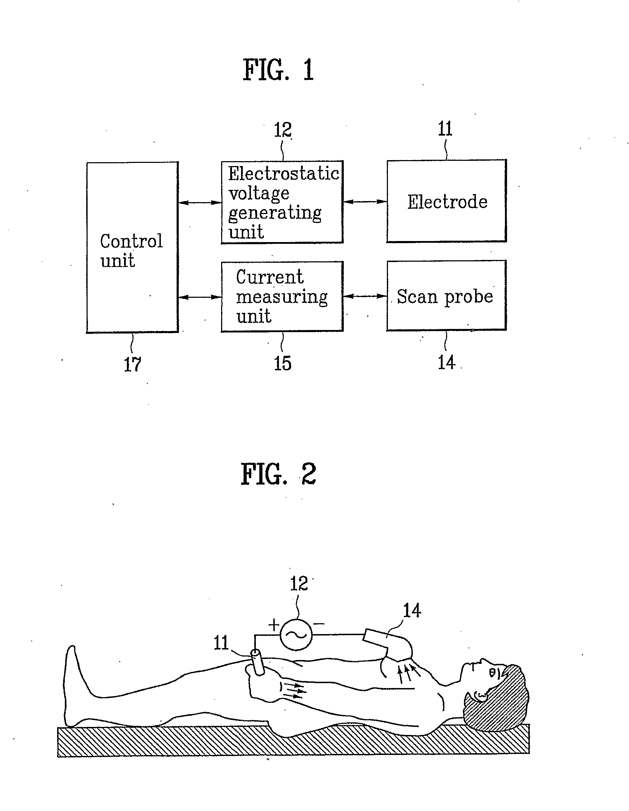

Preferred embodiments of the present invention will be described in detail with reference to the attached drawings. FIG. 1 illustrates a block diagram of an apparatus for detecting a lesion in accordance with a preferred embodiment of the present invention, and FIG. 2 illustrates a diagram showing a method for detecting a lesion by using an apparatus for detecting a lesion in accordance with a preferred embodiment of the present invention.

There is an electrode 11 in contact with a part of a body for applying a voltage to the body. As shown in FIG. 2, the electrode 11 provides the voltage to an inner part of the body through a part of the body, such as a hand.

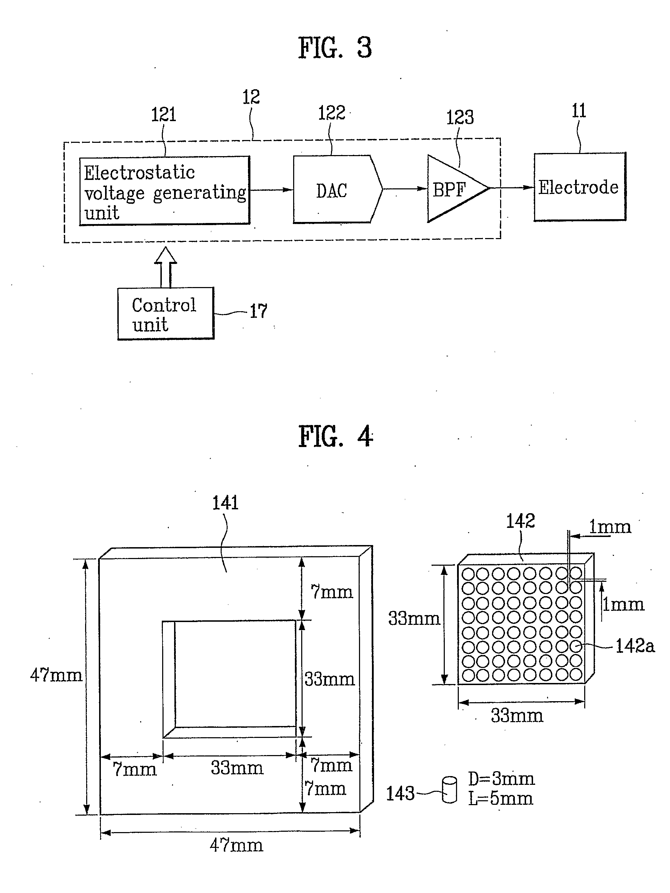

The voltage generating unit 12 supplies the voltage to the electrode 11. The voltage generating unit 12 generates one or more than one particular voltages of sinusoidal waves having frequencies different from each other, and provides to the electrode 11. As shown in FIG. 3, the voltage generating unit 12 includes an FPGA (field programmable gate array) based digital waveform generating unit 121, a digital-analog converter (DAC) 122, and a band pass filter (BPF) 123.

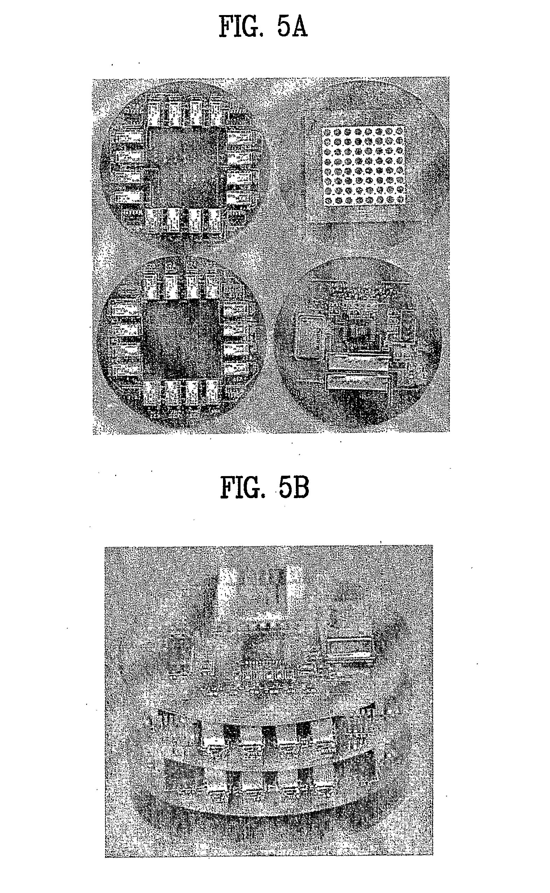

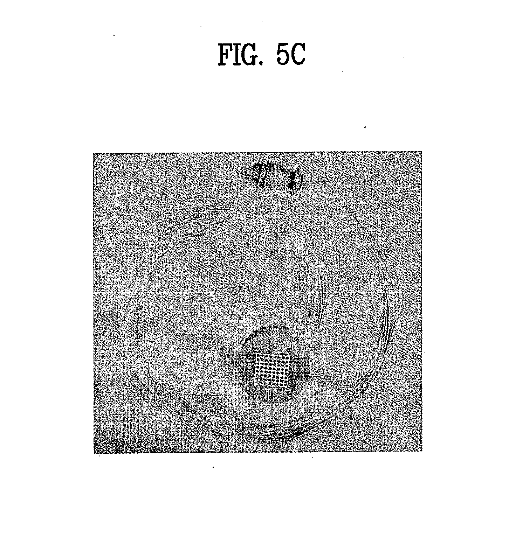

The scan probe 14 is brought into contact with a part of body intended to examine, and senses signals flowing through the part intended to examine, i.e., currents. If it is intended to detect breast cancer, the scan probe 14 scans currents at a breast. The scan probe 14 includes planar array of electrodes. The planar array of electrodes sense the currents flowing through the part intended to examine in a state a reference voltage thereof is maintained. FIG. 4 illustrates a scan probe of the present invention. As shown in FIG. 4, the scan probe 14 includes an array of cylindrical sense electrodes 143 arranged at fixed intervals. For an example, the scan probe 14 includes 8x8, or 16x16 electrodes 143. In order to arrange the electrodes 143 at fixed intervals, a plate 142 for fixedly securing the electrodes 143 is required. The plate 142 has holes 142a for placing the electrodes 143 therein, respectively, and is formed of an insulating material for electrically isolating the electrodes 143. The plate 142 has a metal frame 141 around the plate 142. The metal frame 141 maintains the electrodes 143 and neighborhood thereof at the reference voltage (0V). FIGS. 5A, 5B, and 5C illustrate diagrams each showing a variation of a scan probe of the present invention. The scan probe has sensing electrodes and switches joined together. As shown, the scan probe includes a substrate of the planar array of electrodes of a plurality of the sensing electrodes, a plurality of switch substrates, and a switch control substrate stacked together. The switch substrates and the switch control substrate enable the plurality of sensing electrodes to provide signals through a small number of cables.

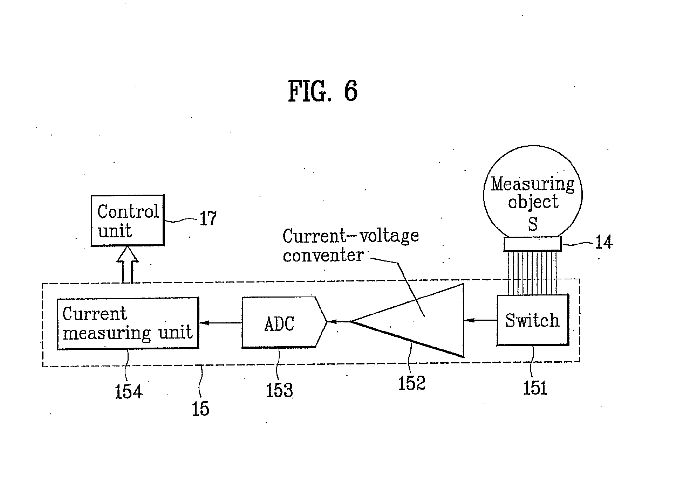

A current measuring unit 15 measures intensities of the currents sensed at the electrodes 143. As shown in FIG. 6, the current measuring unit 15 includes a switch 151 for providing the currents sensed at the electrodes 143 selectively, a current-voltage converter 152 for converting the current from the switch into a voltage, an analog-digital converter (ADC) 153 for converting an analog signal from the current-voltage converter 152 into a digital signal, and a digital phase sensitive current measuring unit 154 for receiving a signal from the analog-digital converter 153, and measuring intensities of fine-currents sensed at the electrodes 143 with reference to the signal.

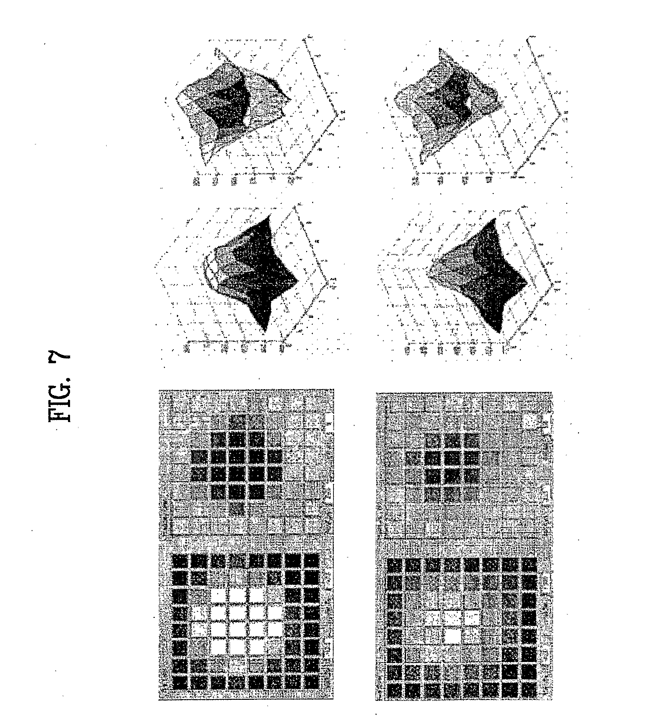

Data on the currents measured at the current measuring unit 154 is provided to the control unit 17, and the control unit 17 detects a real component (magnitude) and an imaginary component (phase) of each of the currents, and calculates a transfer admittance data based thereon. Since the current flows through a lesion, such as a cancer, or tumor tissue, better than a normal tissue, existence of the lesion can be determined by using the transfer admittance data that is proportional to the current intensities. The control unit 17 also can calculate a position and a size of the lesion by using the transfer admittance data, and display an inner part of the examined part in a 3-D image by using the calculated values as shown in FIG. 7. The image provides information on conductivity and permittivity of the organic tissue, such that the user knows, not only existence of cancer, tumor or the like, but also the position and size of the lesion, accurately.

A method for detecting the position and the size of the lesion with reference to the transfer admittance data will be described.

FIRST EMBODIMENT

The control unit 17 calculates the position and the size of the lesion by using the following equation of an algorithm, and makes the lesion into an image by using the data on the position and the size of the lesion. The relation of the transfer admittance data, and the position and the size of the lesion can be defined as the following equation (1).

$$g(x,y) - g_0(x,y) = A \frac{6\alpha(\tau_1 - \tau_2)}{2\tau_1 + \tau_2} \frac{2d^2 - (x-\xi_1)^2 - (y-\xi_2)^2}{4\pi[(x-\xi_1)^2 - (y-\xi_2)^2 + d^2]^{5/2}} \quad \text{-----------------------}$$

-(1)

where,

A denotes a volume of the lesion,
(x,y) denotes any point in a plane $\Gamma$ of the scan probe 14,
g(x,y) denotes a distribution of measured transfer admittances (a function defined in the $\Gamma$),
$\alpha$ denotes an average of the transfer admittance distribution g(x,y) in the scan probe 14 plane $\Gamma$,
$g_0(x,y)$ denotes the transfer admittance distribution when no lesion exists,
d denotes a vertical distance from a point ($\xi_1$, $\xi_2$) in the scan probe 14 plane to a center of gravity of the lesion,
($\xi_1$, $\xi_2$) denotes a pinpoint in the scan probe 14 plane opposite to the lesion in the body,
$\tau_1 = \sigma_1 + j\omega\varepsilon_1$, where $\sigma_1$ denotes an average conductivity of a normal tissue of breast, $\varepsilon_1$ denotes an average permittivity of a normal tissue, and $\omega$ denotes a frequency of a sinusoidal wave,
$\tau_2 = \sigma_2 + j\omega\varepsilon_2$, where $\sigma_2$ denotes an average conductivity of a tumor tissue of breast, and $\varepsilon_2$ denotes an average permittivity of a tumor tissue.

The following equations (2) ~ (4) can be derived from above equation (1), which enables to estimate the position and the size of the lesion with reference to the transfer admittance distribution.

$$(\xi_1, \xi_2) = \frac{\arg\max}{(x,y) \in \Gamma} |g(x,y) - g_0(x,y)| \quad \text{------------------------------------------}$$

(2)

The above equation (2) enables to obtain a pinpoint ($\xi_1$, $\xi_2$) in the scan probe 14 plane $\Gamma$ opposite to the position of lesion (a central point of the lesion) in the body. The pinpoint ($\xi_1$, $\xi_2$) in the scan probe 14 plane $\Gamma$ is a point in the scan probe 14 plane $\Gamma$ at which an absolute value |g(x,y)- $g_0$(x,y)| is the maximum. In order to obtain the pinpoint ($\xi_1$,

$\xi_2$), the control unit 17 compares the measured admittances g(x,y) to the reference admittances $g_0$(x,y), respectively. For an example, if the scan probe 14 has 8x8 sensing electrodes 143, the control unit 17 calculates differences of the admittance values measured at the 64 sensing electrodes 143, and reference admittance values for the 64 sensing electrodes 143, respectively. That is, a difference of a measured admittance value and the reference admittance value is calculated for each of the sensing electrodes 143. Then, one of the sensing electrodes 143 having a maximum value of the difference values is fixed as the pinpoint ($\xi_1$, $\xi_2$) in the scan probe 14 plane opposite to the position of the lesion.

$$|\frac{g(\xi_1,\xi_2)-g_0(\xi_1,\xi_2)}{g(x,y)-g_0(x,y)}|=\frac{|2-\frac{l^2}{d^2}|}{2(\frac{l^2}{d^2}+1)^{5/2}} \quad ------------------------------------------------$$

(3)

The equation (3) enables to obtain a depth of the lesion d. The (x,y) denotes any point in the scan probe 14 plane $\Gamma$ in the neighborhood of the pinpoint ($\xi_1$, $\xi_2$), and $\lambda$ denotes a distance between the point (x,y) and the pinpoint ($\xi_1$, $\xi_2$), $\lambda=\sqrt{(x-\xi_1)^2+(y-\xi_2)^2}$ . If the pinpoint ($\xi_1$, $\xi_2$) in the scan probe 14 plane opposite to the lesion position is obtained from the equation (2), the control unit 17 calculates the distance $\lambda$ between the pinpoint ($\xi_1$, $\xi_2$), and the any point (x,y). Then, the control unit 17 calculates a distance 'd' from the pinpoint ($\xi_1$, $\xi_2$) in the scan probe 14 plane to the lesion with reference to the measured admittance value g($\xi_1$, $\xi_2$), and the reference admittances $g_0$($\xi_1$, $\xi_2$) at the pinpoint ($\xi_1$, $\xi_2$), the measured admittance value g(x,y) and the reference admittances $g_0$(x,y) at the any point (x, y), and the $\lambda$. As can be noted in the equation (3), the distance 'd' is dependent on a ratio of a difference between two admittance values g(x,y)- $g_0$(x,y), and the a difference between the two admittance values g($\xi_1$, $\xi_2$)- $g_0$($\xi_1$, $\xi_2$), as well as $\lambda$. If the pinpoint ($\xi_1$, $\xi_2$) in the scan probe 14 plane opposite to the lesion position, and the distance d from the pinpoint ($\xi_1$, $\xi_2$) to the lesion are known, the position of the lesion in the body can be known, accurately.

$$A=\frac{\pi|2+\frac{\tau_2}{\tau_1}|d^3|g(\xi_1,\xi_2)-g_0(\xi_1,\xi_2)|}{3|1-\frac{\tau_2}{\tau_1}||\alpha|} \quad ------------------------------------------------$$

(4)

Above equation (4) enables to obtain a size(volume) A of the lesion. The $\tau_2/\tau_1$ is a preset ratio of conductivities, or permittivties between the lesion and the normal tissue. Once the distance d from the pinpoint ($\xi_1$, $\xi_2$) in the scan probe 14 plane to the lesion is calculated, the control unit 17 can calculate the volume A of the lesion. As expressed in above equation (4), it can be noted that the volume A of the lesion is calculated with reference to the distance d, the difference g($\xi_1$, $\xi_2$)- $g_0$($\xi_1$, $\xi_2$) of the measured admittance value g($\xi_1$, $\xi_2$), and the reference admittance value $g_0$($\xi_1$, $\xi_2$), and the average value $\alpha$ of the transfer admittance distribution g(x,y) in the scan probe 14 plane.

If it is intended to estimate the position and the size of the lesion actually by using the equation, a value of the reference admittance distribution $g_0$(x,y) is required for a case no lesion exists. The value of the reference admittance distribution $g_0$(x,y) can be obtained by using any one of the following methods.

First, the admittance distribution value of a part symmetry to the part of the body intended to examine is used. For an example, if it is intended to examine a left breast, the admittance distribution measured at the right breast is used as the reference admittance distribution, and vice versa.

Second, an average of the measured admittance distribution g(x, y) is used as the reference admittance distribution $g_0$(x,y).

Third, a statistical value (average value) of the admittance distribution obtained through many experiment without lesions is used as the reference admittance distribution $g_0$(x,y). For an example, in a case if a woman in her 20s is examined for breast cancer, an average of the admittance distribution obtained from women of 20s without breast cancer is used as the reference admittance $g_0$(x,y).

SECOND EMBODIMENT

In order to implement the second embodiment, the electrostatic voltage generating unit 12 generates electrostatic voltages sinusoidal waves having frequencies different from each other, and the electrodes 11 apply the two sinusoidal electrostatic voltages to the body in succession. After detecting transfer admittance distributions of the sinusoidal electrostatic voltages of difference frequencies through the scan probe 14, the control unit 17 calculates a size and a position of the lesion by using the detected transfer admittance distributions.

If the sinusoidal electrostatic voltages of different frequencies are used, it is assumed that the transfer admittance distributions $g(x,y)$, and $\tilde{g}(x,y)$ are for the two sinusoidal electrostatic voltages of different frequencies $\omega$, and $\tilde{\omega}$, and complex conductivities of a normal tissue and a tumor tissue for the frequency $\tilde{\omega}$ are $\tilde{\tau}_1 = \sigma_1 + j\tilde{\omega}\varepsilon_1$, and $\tilde{\tau}_2 = \sigma_2 + j\tilde{\omega}\varepsilon_2$. Then, relation of the transfer admittance distributions for the two sinusoidal electrostatic voltages of different frequencies $\omega$, and $\tilde{\omega}$ and the position and the size of the lesion can be defined as the following equation (5).

$$\tilde{g}(x,y) - g(x,y) = A\frac{18\alpha(\tau_2 - \tilde{\tau}_2)}{(2\tau_1 + \tau_2)(2\tilde{\tau}_1 + \tilde{\tau}_2)}\frac{2d^2 - (x - \xi_1)^2 - (y - \xi_2)^2}{4\pi[(x - \xi_1)^2 - (y - \xi_2)^2 + d^2]^{5/2}} \quad \text{----------}$$

(5)

That is, the electrostatic voltages of different frequencies are applied, and one of the transfer admittance distributions for the electrostatic voltages of different frequencies is used as a reference admittance distribution. An algorithm for detecting the position and the size of the breast cancer with reference to the equation (5) is as follows.

$$(\xi_1, \xi_2) = \frac{\arg\max}{(x,y) \in \Gamma}|\tilde{g}(x,y) - g(x,y)| \quad \text{------------------------------------------------}$$

(6)

The above equation (6) enables to obtain the pinpoint $(\xi_1, \xi_2)$ in the scan probe 14 plane $\Gamma$ opposite to the position of lesion. The pinpoint $(\xi_1, \xi_2)$ in the scan probe 14 plane $\Gamma$ is a point in the scan probe 14 plane $\Gamma$ at which $|\tilde{g}(x,y) - g(x,y)|$ is the maximum. In order to obtain the pinpoint $(\xi_1, \xi_2)$, the control unit 17 compares the admittance distribution $\tilde{g}(x,y)$ for $\tilde{\omega}$ to the admittance difference $g(x,y)$ for $\omega$. Then, a point in the scan probe 14 plane having a maximum value of the differences of the two admittances is fixed as the pinpoint $(\xi_1, \xi_2)$ in the scan probe 14 plane opposite to the lesion position.

$$|\frac{\tilde{g}(\xi_1, \xi_2) - g(\xi_1, \xi_2)}{\tilde{g}(x, y) - g(x, y)}| = \frac{|2 - \frac{l^2}{d^2}|}{2(\frac{l^2}{d^2} + 1)^{5/2}} \quad \text{----------------------------------------------------}$$

(7)

The equation (7) enables to obtain a depth of the lesion d. The (x, y) denotes any point in the scan probe 14 plane $\Gamma$ in the neighborhood of the pinpoint $(\xi_1, \xi_2)$, and $\lambda$ denotes a distance between the any point (x, y) and the pinpoint $(\xi_1, \xi_2)$, $\lambda = \sqrt{(x - \xi_1)^2 + (y - \xi_2)^2}$. The control unit 17 calculates the distance $\lambda$ between the any point (x, y) and the pinpoint $(\xi_1, \xi_2)$, and calculates a distance d from the pinpoint $(\xi_1, \xi_2)$ in the scan probe 14 plane to the lesion with reference to the two admittance values $\tilde{g}(\xi_1, \xi_2)$, and $g(\xi_1, \xi_2)$ measured at the pinpoint $(\xi_1, \xi_2)$, the two admittance values $g(x,y)$, g(x,y) measured at the any point (x, y), and the $\lambda$. As can be noted in the equation (7), the distance d is dependent on a ratio of a difference between two admittance values $\tilde{g}(x,y) - g(x,y)$, and a difference between the two admittance values $\tilde{g}(\xi_1, \xi_2) - g(\xi_1, \xi_2)$, as well as $\lambda$. If the pinpoint $(\xi_1, \xi_2)$ of the lesion in the scan probe 14 plane, and the distance d from the pinpoint $(\xi_1, \xi_2)$ to the lesion are known, the position of the lesion in the body can be known,

accurately.

$$A \equiv \frac{\pi \mid 2\tau_1 + \tau_2 \parallel 2\widetilde{\tau_1} + \widetilde{\tau_2} \mid \widetilde{g}(\xi_1,\xi_2) - g(\xi_1,\xi_2) \mid d^3}{9 \mid \alpha \parallel \tau_2 - \widetilde{\tau_2} \mid}$$

(8)

Above equation (8) enables to obtain a size(volume) A of the lesion. Once the distance d from the pinpoint ($\xi_1$, $\xi_2$) in the scan probe 14 plane to the lesion is calculated, the control unit 17 can calculate the volume A of the lesion. As expressed in above equation (8), it can be noted that the volume A of the lesion is dependent on the distance d, the difference $\widetilde{g}(\xi_1,\xi_2)$ - $g(\xi_1,\xi_2)$ of the two admittance values, and the average value $\alpha$ of the transfer admittance distribution $\widetilde{g}(x,y)$ in the scan probe 14 plane, as well as data $\tau_1$, $\tau_2$, $\widetilde{\tau_1}$, and $\widetilde{\tau_2}$ on the conductivities and permittivities for the two sinusoidal electrostatic voltage.

Industrial Applicability

As has been described, the present invention permits, not only determination of existence of lesion in the body, but also a position and a size of the lesion with reference to admittance data. As a technology for detecting breast cancer, fabrication of an apparatus is possible, which costs lower, and harmless to a human body than the related art apparatus. Accordingly, the present invention can be useful in diagnosis of breast cancer.

**Claims**

1.  An apparatus for detecting a lesion, comprising:

    an electrode for applying two voltages of different first and second frequencies to a part of a body;
    a probe having a plurality of sensing electrodes for sensing signals induced by the two voltages of the different first and second frequencies from an examining part of the body; and
    a control unit for determining a pinpoint of the lesion in a probe plane opposite to a center of the lesion with reference to the sensed signals, calculating a distance between the center of the lesion and the pinpoint of the lesion in the probe plane with reference to the sensed signals, calculating a volume of the lesion with reference to an average of the sensed signals, the distance, an intensity of the signal sensed at the pinpoint of the lesion in the probe plane; and
    determining a position of a lesion in the body with reference to the determined pinpoint and the calculated distance,
    wherein the control unit calculates a difference value between an intensity of a signal of the first frequency and an intensity of a signal of the second frequency of the sensed signals of the sensing electrodes, and determines a position of the sensing electrode which has a maximum value of the calculated difference values as the pinpoint in the probe plane opposite to the center of lesion.

2.  The apparatus as claimed in claim 1 , wherein the probe includes;
    a plate for arranging the sensing electrodes at regular intervals thereon, and
    a metal frame around the plate for maintaining the sensing electrodes at a reference voltage.

3.  The apparatus as claimed in claim 1, further comprising an electrostatic voltage generating unit for providing electrostatic voltages of sinusoidal waves having the first and second frequencies different from each other.

$$(\xi_1,\xi_2) = \frac{\arg\max}{(x,y) \in \Gamma} \mid \widetilde{g}(x,y) - g(x,y) \mid$$

$$\widetilde{g}(x,y) - g(x,y) = A \frac{18\alpha(\tau_2 - \widetilde{\tau_2})}{(2\tau_1 + \tau_2)(2\widetilde{\tau_1} + \widetilde{\tau_2})} \frac{2d^2 - (x - \xi_1)^2 - (y - \xi_2)^2}{4\pi[(x - \xi_1)^2 - (y - \xi_2)^2 + d^2]^{5/2}}$$

$$\left|\frac{\widetilde{g}(\xi_1,\xi_2)-g(\xi_1,\xi_2)}{\widetilde{g}(x,y)-g(x,y)}\right|=\frac{\left|2-\dfrac{l^2}{d^2}\right|}{2(\dfrac{l^2}{d^2}+1)^{5/2}}$$

4. The apparatus as claimed in claim 1 , wherein the control unit determines the pinpoint of the lesion in the probe plane as the following equation

$$(\xi_1,\xi_2)=\frac{\arg\max}{(x,y)\in\Gamma}\,|\,\widetilde{g}(x,y)-g(x,y)\,|$$

where,

$(\xi_1, \xi_2)$ denotes the pinpoint,
$\Gamma$ denotes the probe plane,
$g(x, y)$ denotes a transfer admittance distribution of the voltage of the first frequency $\omega$,
$\widetilde{g}(x,y)$ denotes a transfer admittance distribution of the voltage of the second frequency $\widetilde{\omega}$,
$(x, y)$ denotes any point in the probe plane in the neighborhood of the pinpoint,
$\widetilde{g}(x,y) - g(x,y)$ is defined as the following equation

$$\widetilde{g}(x,y)-g(x,y)=A\frac{18\alpha(\tau_2-\widetilde{\tau}_2)}{(2\tau_1+\tau_2)(2\widetilde{\tau}_1+\widetilde{\tau}_2)}\frac{2d^2-(x-\xi_1)^2-(y-\xi_2)^2}{4\pi[(x-\xi_1)^2-(y-\xi_2)^2+d^2]^{5/2}}$$

$\alpha$ denotes an average of the transfer admittance distribution $g(x,y)$ in the probe plane,
$\tau_1 = \sigma_1 + j\omega\varepsilon_1$,
$\sigma_1$ denotes an average conductivity of a normal tissue of the examining part of the body for the first frequency,
$\varepsilon_1$ denotes an average permittivity of the normal tissue,
$\tau_2 = \sigma_2 + j\omega\varepsilon_2$,
$\sigma_2$ denotes an average conductivity of a tumor tissue of the examining part of the body for the first frequency,
$\varepsilon_2$ denotes an average permittivity of the tumor tissue.
$\widetilde{\tau}_1 = \sigma_1 + j\widetilde{\omega}\varepsilon_1$, and $\widetilde{\tau}_2 = \sigma_2 + j\widetilde{\omega}\varepsilon_2$ denote complex conductivities of a normal tissue and a tumor tissue of the examining part of the body for the second frequency, respectively,
A denotes the volume of the lesion, and
d denotes the distance between the center of the lesion and the pinpoint of the lesion; and
wherein the distance d is calculated as the following equation,

$$\left|\frac{\widetilde{g}(\xi_1,\xi_2)-g(\xi_1,\xi_2)}{\widetilde{g}(x,y)-g(x,y)}\right|=\frac{\left|2-\dfrac{l^2}{d^2}\right|}{2(\dfrac{l^2}{d^2}+1)^{5/2}}$$

where $l$ denotes a distance between the any point (x, y) and the pinpoint $(\xi_1, \xi_2)$.

**Patentansprüche**

1. Vorrichtung zum Erkennen einer Läsion, umfassend:

eine Elektrode zum Anlegen von zwei Spannungen mit voneinander unterschiedlicher erster und zweiter Frequenz an einem Teil eines Körpers;

eine Sonde mit einer Vielzahl von Messelektroden zum Messen von Signalen, welche durch die zwei Spannungen mit der voneinander unterschiedlicher erster und zweiter Frequenz an einem zu untersuchenden Teil des Körpers verursacht werden; und

eine Steuereinheit zum Bestimmen eines Zielpunkts der Läsion in einer Untersuchungsebene gegenüber eines Zentrums der Läsion mit Bezug auf die gemessenen Signale, Berechnen einer Distanz zwischen dem Zentrum der Läsion und

dem Zielpunkt der Läsion in der Untersuchungsebene mit Bezug auf die gemessenen Signale, Berechnen eines Volumens der Läsion mit Bezug auf einen Mittelwert der gemessenen Signale, die Distanz, eine Intensität des am Zielpunkt der Läsion in der Untersuchungsebene gemessenen Signals; und

Bestimmen einer Position einer Läsion in dem Körper mit Bezug auf den bestimmten Zielpunkt und die berechnete Distanz,

wobei die Steuereinheit einen Differenzwert zwischen einer Intensität eines Signal der ersten Frequenz und einer Intensität eines Signals der zweiten Frequenz der gemessenen Signale der Messelektroden berechnet und eine Position der Messelektrode bestimmt,

welche einen Maximalwert der berechneten Differenzwerte als Zielpunkt in der Untersuchungsebene gegenüber dem Zentrum der Läsion hat.

2. Vorrichtung gemäß Anspruch 1, wobei die Sonde umfasst;
eine Platte um darauf die Messelektroden in regelmäßigen Abständen anzuordnen, und ein Metallrahmen um die Platte um die Messelektroden auf einer Referenzspannung zu halten.

3. Vorrichtung gemäß Anspruch 1, ferner umfassend eine elektrostatische Spannungserzeugende Einheit zum Bereitstellen von elektrostatischen Spannungen von sinusoidalen Wellen mit der voneinander unterschiedlichen ersten und zweiten Frequenz.

4. Vorrichtung gemäß Anspruch 1, wobei die Steuereinheit den Zielpunkt der Läsion in der Untersuchungsebene als folgende Gleichung bestimmt

$$(\overset{..}{\xi}_1, \xi_2) = \underset{(x,y)\in\Gamma}{\arg\max} |\tilde{g}(x,y) - g(x,y)|$$

wobei

$(\xi_1, \xi_2)$ den Zielpunkt bezeichnet,
$\Gamma$ die Untersuchungsebene bezeichnet,
$g(x,y)$ eine Transadmittanzverteilung der Spannung der ersten Frequenz $\omega$ bezeichnet,
$\tilde{g}(x,y)$ eine Transadmittanzverteilung der Spannung der zweiten Frequenz $\tilde{\omega}$ bezeichnet,
$(x,y)$ beliebigen Punkt in der Untersuchungsebene in der Nachbarschaft des Zielpunkts bezeichnet,
$\tilde{g}(x,y) - g(x,y)$ als folgende Gleichung definiert ist:

$$\tilde{g}(x,y) - g(x,y) = A \frac{18\alpha(\tau_2 - \tilde{\tau}_2)}{(2\tau_1 + \tau_2)(2\tilde{\tau}_1 + \tilde{\tau}_2)} \frac{2d^2 - (x - \xi_1)^2 - (y - \xi_2)^2}{4\pi[(x - \xi_1)^2 - (y - \xi_2)^2 + d^2]^{5/2}}$$

$\alpha$ einen Mittelwert der Transadmittanzverteilung g(x,y) in der Untersuchungsebene bezeichnet,

$$\tau_1 = \sigma_1 + j\omega\varepsilon_1,$$

$\sigma_1$ eine mittlere Leitfähigkeit eines normalen Gewebes des zu untersuchenden Teils des Körpers für die erste Frequenz bezeichnet,
$\varepsilon_1$ eine mittlere Dielektrizitätskonstante des normalen Gewebes bezeichnet,

$$\tau_2 = \sigma_2 + j\omega\varepsilon_2,$$

$\sigma_2$ eine mittlere Leitfähigkeit eines Tumor-Gewebes des zu untersuchenden Teils des Körpers für die erste Frequenz bezeichnet,

$\varepsilon_2$ eine mittlere Dielektrizitätskonstante des Tumor-Gewebes bezeichnet,

$\tilde{\tau}_1 = \sigma_1 + j\tilde{\omega}\varepsilon_1$ und $\tilde{\tau}_2 = \sigma_2 + j\tilde{\omega}\varepsilon_2$ komplexe Leitfähigkeiten eines normalen Gewebes bzw. eines Tumor-Gewebes des zu untersuchenden Teils des Körpers für die zweite Frequenz bezeichnen,

$A$ das Volumen der Läsion bezeichnet,

d die Distanz zwischen dem Zentrum der Läsion und dem Zielpunkt der Läsion bezeichnet und

wobei die Distanz d als folgende Gleichung berechnet wird,

$$\left| \frac{\tilde{g}(\xi_1, \xi_2) - g(\xi_1, \xi_2)}{\tilde{g}(x, y) - g(x, y)} \right| = \frac{\left| 2 - \dfrac{l^2}{d^2} \right|}{2 \left( \dfrac{l^2}{d^2} + 1 \right)^{5/2}}$$

wobei $l$ eine Distanz zwischen dem beliebigen Punkt *(x, y)* und dem Zielpunkt $(\xi_1, \xi_2)$ bezeichnet.

## Revendications

1. Appareil pour détecter une lésion, comprenant :

   une électrode pour appliquer deux tensions de première et seconde fréquences différentes sur une partie d'un corps ;
   une sonde comportant une pluralité d'électrodes détectrices pour détecter des signaux induits par les deux tensions des première et seconde fréquences différentes à partir d'une partie d'examen du corps ; et
   une unité de commande pour déterminer un point de localisation de la lésion dans un plan de sonde opposé à un centre de la lésion par rapport aux signaux détectés, calculer une distance entre le centre de la lésion et le point de localisation de la lésion dans le plan de sonde par rapport aux signaux détectés, calculer un volume de la lésion par rapport à une moyenne des signaux détectés, la distance, une intensité du signal détecté au point de localisation de la lésion dans le plan de sonde ; et
   déterminer une position d'une lésion dans le corps par rapport au point de localisation déterminé et à la distance calculée,
   dans lequel l'unité de commande calcule une valeur de différence entre une intensité d'un signal de la première fréquence et une intensité d'un signal de la seconde fréquence des signaux détectés des électrodes détectrices, et détermine une position de l'électrode détectrice qui possède une valeur maximum des valeurs de différence calculées en tant que point de localisation dans le plan de sonde opposé au centre de lésion.

2. Appareil selon la revendication 1, dans lequel la sonde comprend :

   une plaque pour agencer les électrodes détectrices à des intervalles réguliers sur celle-ci, et
   un cadre métallique autour de la plaque pour maintenir les électrodes détectrices à une tension de référence.

3. Appareil selon la revendication 1, comprenant en outre une unité génératrice de tension électrostatique pour fournir des tensions électrostatiques d'ondes sinusoïdales possédant les première et seconde fréquences différentes l'une de l'autre.

4. Appareil selon la revendication 1, dans lequel l'unité de commande détermine le point de localisation de la lésion dans le plan de sonde selon l'équation suivante

$$(\xi_1, \xi_2) = \frac{\arg \max}{(x,y) \in \Gamma} | \tilde{g}(x,y) - g(x,y) |$$

où,

$(\xi_1, \xi_2)$ dénote le point de localisation,

$\Gamma$ dénote le plan de sonde,

$g(x,y)$ dénote une distribution d'admittance de transfert de la tension de la première fréquence $\omega$,

$\tilde{g}(x,y)$ dénote une distribution d'admittance de transfert de la tension de la seconde fréquence $\tilde{\omega}$,

$(x,y)$ dénote un quelconque point dans le plan de sonde dans le voisinage du point de localisation,

$\tilde{g}(x,y) - g(x,y)$ est défini selon l'équation suivante :

$$\tilde{g}(x,y) - g(x,y) = A \frac{18\alpha(\tau_2 - \tilde{\tau}_2)}{(2\tau_1 + \tau_2)(2\tilde{\tau}_1 + \tilde{\tau}_2)} \frac{2d^2 - (x - \xi_1)^2 - (y - \xi_2)^2}{4\pi[(x - \xi_1)^2 - (y - \xi_2)^2 + d^2]^{5/2}}$$

$\alpha$ dénote une moyenne de la distribution d'admittance de transfert $g(x,y)$ dans le plan de sonde,

$$\tau_1 = \sigma_1 + j\omega\varepsilon_1,$$

$\sigma_1$ dénote une conductivité moyenne d'un tissu normal de la partie d'examen du corps pour la première fréquence,

$\varepsilon_1$ dénote une permittivité moyenne du tissu normal,

$$\tau_2 = \sigma_2 + j\omega\varepsilon_2,$$

$\sigma_2$ dénote une conductivité moyenne d'un tissu tumoral de la partie d'examen du corps pour la première fréquence,

$\varepsilon_2$ dénote une permittivité moyenne du tissu tumoral,

$\tilde{\tau}_1 = \sigma_1 + j\tilde{\omega}\varepsilon_1$ et $\tilde{\tau}_2 = \sigma_2 + j\tilde{\omega}\varepsilon_2$ dénotent des conductivités complexes d'un tissu normal et d'un tissu tumoral de la partie d'examen du corps pour la seconde fréquence, respectivement,

A dénote le volume de la lésion, et

d dénote la distance entre le centre de la lésion et le point de localisation de la lésion ; et

dans lequel la distance d est calculée selon l'équation suivante,

$$\left| \frac{\tilde{g}(\xi_1, \xi_2) - g(\xi_1, \xi_2)}{\tilde{g}(x,y) - g(x,y)} \right| = \frac{|2 - \frac{l^2}{d^2}|}{2(\frac{l^2}{d^2} + 1)^{5/2}},$$

où $l$ dénote une distance entre l'un quelconque point $(x,y)$ et le point de localisation $(\xi_1, \xi_2)$.

# FIG. 1

# FIG. 2

## FIG. 3

## FIG. 4

FIG. 5A

FIG. 5B

# FIG. 5C

# FIG. 6

FIG. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2003004432 A1
- US 2002193685 A1
- US 2002183645 A1
- US 5810742 A